Europäisches Patentamt

European Patent Office

Office européen des brevets

(11) Publication number: **0 302 717**
A1

# EUROPEAN PATENT APPLICATION

(21) Application number: **88307177.1**

(22) Date of filing: **03.08.88**

(51) Int. Cl.⁴: **A 61 F 2/30**
      **A 61 L 27/00**

(30) Priority: **14.03.88 GB 8805979**
              **04.08.87 GB 8718434**

(43) Date of publication of application:
      **08.02.89 Bulletin 89/06**

(84) Designated Contracting States: **DE FR GB IT**

(71) Applicant: **ION TECH LIMITED**
      **2 Park Street Teddington**
      **Middlesex TW11 0LT (GB)**

(72) Inventor: **Franks, Joseph**
      **Ion Tech Limited 2 Park Street**
      **Teddington Middlesex, TW11 0LT (GB)**

(74) Representative: **Cline, Roger Ledlie et al**
      **EDWARD EVANS & CO. Chancery House 53-64 Chancery**
      **Lane**
      **London WC2A 1SD (GB)**

(54) **Body implant.**

(57) A member 10 for implanting in the human body comprises a core coated with hard carbon which is preferably diamond-like carbon. The core may be a titanium alloy or high density polyethylene.

EP 0 302 717 A1

**Description**

## BODY IMPLANT

This invention relates to a member for implanting in the human body. It must not harm the body in which it is implanted, nor must it be rejected by the body.

The present invention provides a member for implanting in the human body, comprising a core coated with hard carbon. This may include amorphous carbon with hydrogen, or ion- bombarded carbon or diamond-like carbon (DLC) which has been shown to be a carbon hydrogen polymer in which the bonding is largely diamond tetragonal (SP$^3$) with some graphitic trigonal (SP$^2$) bonding. The films are hard, abrasion resistant, chemically inert, impervious and adhere strongly to a variety of substrates. Because of their transparency in the infra-red, an important application has so far been anti-reflective protective coatings for infra-red optics. Hard carbon has been found to be compatible with biological environments. The advantage of using hard carbon to coat inserts or prostheses for bio engineering or medical purposes is to avoid rejection and to protect the inserts from attack by the environment and the environment from deleterious effects from the inserts.

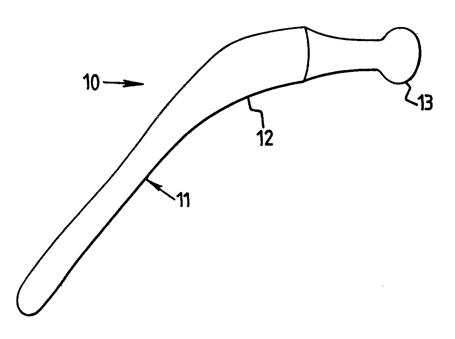

An example of the invention will now be described with reference to the accompanying figure, which is a diagrammatic representation of an artificial hip joint. The joint 10 is made of titanium alloy having 6% aluminium and 4% vanadium and has an elongated first portion 11 of approximately circular cross section whose diameter increases towards one end 12 where it bends through about 30° and is formed with a ball joint 13. This shape of hip joint 10 is conventional. Such joints may be fabricated from metals, ceramics and polymeric materials, based on carbon, silicon and carbon-silicon compounds.

The joint member 10 is according to this embodiment coated with a hard carbon such as DLC. GB-A-2 114 963 describes a method of producing coatings of hard carbon. Experiments have verified in vivo and in vitro that members so coated are non-toxic, better protected from chemical attack and provide surfaces which are more resistant to mechanical wear, besides promoting cell attachment and discouraging blood clotting. Immersion of such a member 10 in a saline solution for two weeks produced no evidence of corrosion. Such coated core members 10 are particularly useful for body inserts, since the corrosion resistance and wear resistance gives the insert member a longer useful life before further replacement is required. A coating thickness of 500 Angstroms is preferred although thicknesses up to 2000 Angstroms may be provided for increased protection.

To test biological compatibility, mouse peritoneal macrophages were seeded on plates half coated with DLC. A noticeable increase in cell density became evident after 3 hours on the coated areas of all plates compared with the uncoated areas. Slow release of lactate hydrogenase up to 48 hours indicated that none of the coatings caused any loss of cell integrity.

As well as adhering strongly to titanium, DLC adheres to ultra high molecular weight polyethylene with a bond strength of 78kg/cm$^2$. A plastic catheter coated with DLC could be bent through 90° without the film cracking.

The hard carbon coating can be produced using a saddle field source as described in the earlier patent specification, or by chemical vapour deposition, for example. DLC films produced by direct beam deposition from a saddle field source from hydrocarbon precursor gases such as propane, butane, and acetylene are compatible with biological environments, adhere to a variety of metal and plastic substrates, protect substrates from the biological environment and the environment from contamination by the substrate.

**Claims**

1. A member for implanting in the human body comprising a core coated with hard carbon.

2. A member as claimed in claim 1 wherein the coating has a thickness in the range 500 - 2000 Amgstroms.

3. A member as claimed in claim 1 or claim 2 wherein the hard carbon comprises diamond-like carbon.

4. A member as claimed in any one of claims 1 to 3 wherein the core comprises a titanium alloy.

5. A member as claimed in any one of claims 1 to 3 wherein the core comprises high molecular weight polyethylene.

6. A member for implanting in the human body substantially as herein before described.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | US-A-4 693 760 (SIOSHANSI)<br>* Abstract; column 2, lines 9-12 *<br>--- | 1,3,4,6 | A 61 F 2/30<br>A 61 L 27/00 |
| Y | GB-A-2 128 637 (TECHNION R & D FOUNDATION LTD)<br>* Page 1, lines 22-30 *<br>----- | 1,3,4,6 | |

|  |  |  | TECHNICAL FIELDS SEARCHED (Int. Cl.4) |
|---|---|---|---|
|  |  |  | A 61 F<br>A 61 L<br>C 23 C |

The present search report has been drawn up for all claims

| Place of search | Date of completion of the search | Examiner |
|---|---|---|
| THE HAGUE | 07-11-1988 | ARGENTINI A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P0401)